(19) Europäisches Patentamt
European Patent Office
Office européen des brevets

(11) Veröffentlichungsnummer: **0 158 880 B1**

(12) # EUROPÄISCHE PATENTSCHRIFT

(45) Veröffentlichungstag der Patentschrift: **04.12.91**

(51) Int. Cl.5: **A61K 9/50**, A61K 39/35

(21) Anmeldenummer: **85103651.7**

(22) Anmeldetag: **27.03.85**

Die Akte enthält technische Angaben, die nach dem Eingang der Anmeldung eingereicht wurden und die nicht in dieser Patentschrift enthalten sind.

(54) **Liposomen mit inhalativen Allergenen zur Behandlung von Allergien, Verfahren zu ihrer Herstellung und diese enthaltende Arzneimittel.**

(30) Priorität: **05.04.84 DE 3412793**

(43) Veröffentlichungstag der Anmeldung:
**23.10.85 Patentblatt 85/43**

(45) Bekanntmachung des Hinweises auf die Patenterteilung:
**04.12.91 Patentblatt 91/49**

(84) Benannte Vertragsstaaten:
**AT BE CH DE FR GB IT LI LU NL SE**

(56) Entgegenhaltungen:
**EP-A- 0 017 557**
**CH-A- 462 383**

**JOURNAL OF ALLERGY AND CLINICAL IMMUNOLOGY, Band 73, Nr. 1, Teil 2, 1984, Seite 118; W. WAGNER et al.: "Immunotherapy with allergen sequestered in liposomes"**

**CHEMICAL ABSTRACTS, Band 98, Nr. 15, 11. April 1983, Seite 191, Nr. 120818t, Columbus, Ohio, US; S.L. COLBY et al.: Liposomes containing 3-n-pentadecylcatechol induce tolerance to toxicodendron"**

(73) Patentinhaber: **Alergia e Inmunologia Abello, S.A.**
**Julian Camarillo 8**
**Madrid 17(ES)**

(72) Erfinder: **Kompa, Hannes Edgar**
**Talfeldstrasse 44**
**W-7950 Biberach 1(DE)**

(74) Vertreter: **Vossius & Partner**
**Siebertstrasse 4 P.O. Box 86 07 67**
**W-8000 München 86(DE)**

**EP 0 158 880 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

BIOLOGICAL ABSTRACTS, Band 76, Nr. 9, 1983, Nr. 61801, BIOLOGICAL ABSTRACTS INC., Philadelphia, P.A., US; V.N. FEDOSEEVA et al.: "Properties of Neisseria perflava liposomal allergens"

BIOLOGICAL ABSTRACTS, Band 74, Nr. 6, 1982, Nr. 36608, BIOLOGICAL ABSTRACTS INC., Philadelphia, P.A., US; HIKARU, NAGATOMI et al.: "Suppressive effect of protein antigen-containing liposomes on immunoglobulin E antibody in the mouse" & JPN J ALLERGOL 30(12): 1099-1105. 1981(RECD. 1982).

**Beschreibung**

Die Erfindung betrifft Liposomen mit inhalativen Allergenen zur Behandlung von Allergien, Verfahren zu ihrer Herstellung und diese enthaltende Arzneimittel.

Werden Lecithine, die gegebenenfalls vorher mit Cholesterinen vermischt wurden, in einem organischen Lösungsmittel gelöst, so bildet sich bekanntlich nach dem Verdampfen des Lösungsmittels eine Lipid-Micell-Schicht auf dem Glasgefäß. Schüttelt oder behandelt man diese Lipide in Gegenwart von Wasser mit Ultraschall, so bilden sich Bilayervesikel entsprechend der Figur 1, die einen Querschnitt durch einen solchen Bilayervesikel darstellt und in der (a) ein Lipidbilayer und (b) wasserlösliche Moleküle bedeuten. Es bilden sich konzentrische, in sich geschlossene Vesikel, Liposomen genannt, mit bimolekularen Schalen, die im Innern, d.h. in ihrem Kern und zwischen den Schalen, wässrige Kompartimente aufweisen. Hierbei enthalten die wässrigen Kompartimente auch die Substanzen, die in dem Wasser vorher bereits gelöst waren, das bei der Herstellung der Liposomen verwendet wurde. Es war also bekannt, Substanzen, beispielsweise Wirkstoffe, in die Liposomen einzuschließen, wobei diese Stoffe niedrig-molekular, wie dies beispielsweise bei vielen Chemotherapeutika der Fall ist, oder aber auch hochmolekular (wie z.B. bei Proteinen) sein können.

Cholesterin ist nicht unbedingt zur Liposomenbildung notwendig, die Einarbeitung von Cholesterin erbringt aber stabilere Liposomenmembranen.

Die Herstellung von Liposomen wird unter anderem beschrieben von O. Zumbuehl und H. G. Weder, Biochim. et Biophys. Acta, 640 (1981), S. 252-262, oder von G. Gregoriadis, D. Putman, L. Louis und D. Neerunjun, J. Biochem. 140, (1974), S. 323-330.

In der Vergangenheit wurden viele Substanzen in Liposomen eingeschlossen, wie z.B. Insulin, Zytostatika, Enzyme, Vitamine, chelatbildende Substanzen. Es wurden aber auch Kontaktallergene auf diese Art und Weise verkapselt, vgl. S.L. Colby et al., J. Invest. Derm. 80, 145-149, 1983. Diese Verkapselung in Liposomen hatte zum Ziel, eingeschlossene Substanzen an bestimmte Empfänger im tierischen oder menschlichen Körper zu bringen; hierzu markierte man beispielsweise die Liposomenmembran mit Antikörpern gegen ein bestimmtes Gewebe. Die Verkapselung in Liposomen wurde aber auch als eine Schutzmaßnahme gewählt, um Substanzen vor einer vorzeitigen Metabolisierung zu bewahren oder auch um die Toxizität bestimmter Substanzen zu kaschieren.

Wenn zur Allergie neigende Menschen inhalative Allergene wie Baum-, Kräuter- und Graspollen, oder Epithelien von Tieren einatmen, so entwickeln sie gegen diese Allergene Antikörper. Diese Antikörper sind vom IgE-Typ und finden sich an Mastzellen oder basophilen Zellen. Trifft ein Allergen auf den Antikörper vom IgE-Typ, so führt dies, gemäß dem nachfolgenden Schema, zu einer Ausschüttung von Histamin, das eine entsprechende allergische Symptomatik, wie Asthma, Rhinitis oder Konjunktivitis, auslöst.

Schema der Histaminfreigabe:

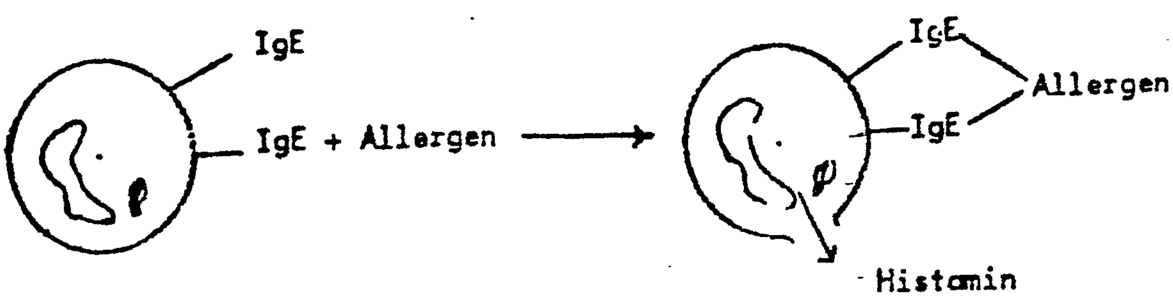


Viele Menschen reagieren beispielsweise sehr empfindlich auf Dermatophagoides farinae, Dermatophagoides pteronyssinus, Gerste, Weide, Hasel, Erle (und Mischung), Beifuß, Gänsefuß, Glaskraut, Spitzwegerich, Sauerampfer (und deren Mischungen), Knäuelgras, Wiesenschwingel, Raygras, Wiesenrispengras, Honiggras, Wiesenlieschgras, Rauchgras, Hundzahngras (und deren Mischungen), Schimmelpilze, Hefen, Bienen- und Wespengift, Küchenschabe, Epithelien von Hund, Katze, Pferd, Meerschweinchen und Schaf.

Die Therapie solcher Allergien aufgrund inhalativer Allergene (IgE-vermittelte Anaphylaxie) besteht bis heute darin, daß man dem Patienten kleine, suballergische Mengen des Allergens in der Absicht zuführt, ihn im weitesten Sinne unempfindlich gegenüber dem Allergen zu machen.

Das Allergen kann dem Patienten subkutan oder per os verabreicht werden. Bei oraler Einnahme ist zu

beachten, daß manche Allergene im Magen-Darmtrakt zerstört werden.

Bei beiden Applikationsformen (subkutan und oral) sind folgende Nachteile in Kauf zu nehmen:

1.) Die Zuführung von nativen Allergenen führt gern zu Nebenwirkungen;

2.) Die orale Therapie ist wegen unvollständiger Resorption und wegen Digestion des Allergens oft wirkunglos.

3.) Die subkutane Injektion schafft Kompliance-Probleme, die die Effizienz der Therapie beeinflussen.

4.) Bei der subkutanen Deponie eines hochlöslichen Allergens ist es oft zweifelhaft, ob dieses zu den Zielzellen (immunkompetente Zellen) gelangt und dort aufgenommen wird. Die erzielte Immunogenität ist damit oftmals gering bzw. ganz in Frage gestellt.

Es ist das Ziel vorliegender Erfindung, für inhalative Allergene oder Allergenfraktionen (major allergens) einen Weg zu finden, die Hyposensibilisierungstherapie sicherer, wirksamer und bequemer zu machen.

Dieses Ziel wurde erfindungsgemäß dadurch erreicht, daß inhalative Allergene oder Allergenfraktionen in die wässrigen Kompartimente von Liposomen eingeschlossen werden, die erhalten werden aus

a) Diacylglycerinphosphatidylethanolaminen mit 8 bis 24 Kohlenstoffatomen in der aliphatischen Acylgruppe und/oder Lecitinen bzw. Lecithinderivaten der allgemeinen Formel

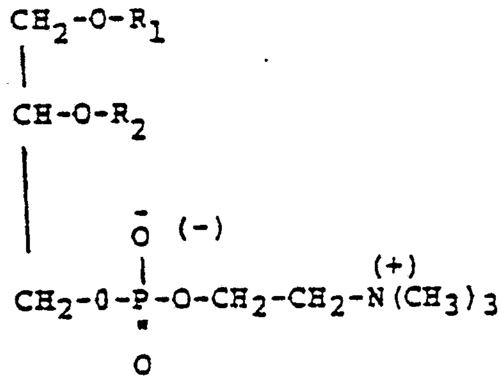

, (I)

in der $R_1$ und $R_2$ aliphatische Acylreste mit 8 bis 24 Kohlenstoffatomen darstellen, wobei $R_2$ aber auch eine Alkylgruppe mit 1 bis 8 Kohlenstoffatomen bedeuten kann, und in der die Gruppen $-OR_1$, $-OR_2$ und $-O-PO_3CH_2-CH_2-N(CH_3)_3$ gegeneinander vertauscht sein können, gegebenenfalls in Anwesenheit von Diacylglycerinphosphorsäureestern mit 8 bis 24 Kohlenstoffatomen im aliphatischen Acylrest und/oder von adjuvanten Lecithinen der allgemeinen Formel I, in der $R_1$ oder $R_2$ ein Wasserstoffatom ist und der andere dieser Reste $R_1$ bzw. $R_2$ einen aliphatischen Acylrest mit 8 bis 24 Kohlenstoffatomen oder einen Alkylrest mit 8 bis 24 Kohlenstoffatomen bedeutet, und

b) Cholesterin, [enthaltend in ihren wässrigen Kompartimenten inhalative Allergene oder Allergenfraktionen in einem isotonen Phosphatpuffer] wobei das Gemisch aus den Lecithinen und dem Cholesterin während der Herstellung der Liposomen einem Druck bis zu 1500 bar ausgesetzt wird.

Durch die dadurch erzielte Verkapselung der Allergene oder Allergenfraktionen ist ein direkter Kontakt zum IgE basophiler Zellen nicht möglich, da sich das Allergen im wässrigen Kompartiment der Liposomen befindet.

Was aber in überraschender Weise zu einer bezüglich ihrer Wirkung gesteigerten Hyposensibilisierungstherapie beiträgt, ist der Befund, daß das mit den inhalativen Allergenen befrachtete Liposom bevorzugt von den Zellen des retikuloendothelialen Systems aufgenommen wird, die auch die entsprechende Immunantwort aufbereiten. Als weiteres überraschendes Moment muß zudem angesehen werden, daß das mit dem Allergen behaftete Liposom auch oral appliziert werden kann, wobei es dann von den Peyerschen Plaques aufgenommen und durch die dränierenden lymphatischen Gefäße in den Kreislauf entlassen wird.

Zusammenfassend läßt sich sagen, daß diese die inhalativen Allergene oder Allergenfraktionen enthaltenden Liposomen diese Allergene bzw. Allergenfraktionen besser resorbierbar machen und die immunkompetente Zellen (z.B. Phagozyten) zu einer adäquaten Antwort (Erzeugung von blockierenden Antikörpern bzw. Toleranz) veranlassen. Ein direkter Kontakt zum IgE von basophilen Zellen bzw. Granulozyten und Mastzellen wird durch die Einkoppelung der Allergene in die wässrigen Kompartimente der Liposomen unterbunden. Während also Allergene beim Menschen und bei Tieren mit allergischer Veranlagung die Bildung von anaphylactoiden Immunglobulinen vom IgE-Typ induzieren, wird durch ihre Inkorporierung in den inneren Kompartimenten der Liposomen diese sepzifische immunogene Eigenschaft überraschenderweise dahingehend geändert, daß jetzt blockierende Immunglobuline vom IgG-Typ entstehen.

Die inhalativen Allergene werden in Liposomen inkorporiert, die aus Diacylglycerinphosphatidylcholinen (Lecithine) Lecitinabkömmlingen oder Diacylglycerinphosphatidylethanolaminen mit 8 bis 24 Kohlenstoffatomen in der aliphatischen Acylgruppe und Cholesterin bestehen. Die Lecithine und Lecithinabkömmlinge besitzen folgende allgemeine Formel I

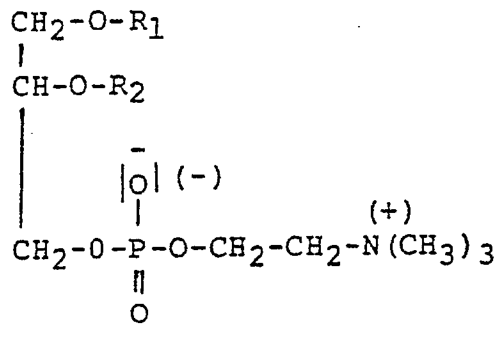

, (I)

in der $R_1$ und $R_2$ aliphatische Acylreste mit 8 bis 24 Kohlenstoffatomen darstellen, wobei $R_2$ aber auch eine Alkylgruppe mit 1 bis 8 Kohlenstoffatomen bedeuten kann, und in der die Gruppen $-OR_1$, $-OR_2$ und $-O-PO_3CH_2-CH_2-N(CH_3)_3$ gegeneinander vertauscht sein können. Es können aber auch sogenannte adjuvante Lecithine der allgemeinen Formel I mitverwendet werden, also Verbindungen der allgemeinen Formel I, in der $R_1$ oder $R_2$ ein Wasserstoffatom ist und der andere dieser Reste $R_1$ bzw. $R_2$ einen aliphatischen Acylrest mit 8 bis 24 Kohlenstoffatomen oder einen Alkylrest mit 8 bis 24 Kohlenstoffatomen darstellt.

Verbindungen der allgemeinen Formel I, worin $R_2$ eine Alkylgruppe darstellt, sind deshalb von Interesse, weil sie, beispielsweise als Bestandteile in einem Gemisch mit Lecithinen, eine verzögernde Wirkung auf den Abbau der Lecithine, insbesondere im Gastrointestinaltrakt, ausüben.

Durch die Art der Substituenten $R_1$ und $R_2$ läßt sich die Phagozytose und die Immnunantwort beeinflussen. Neben reinen Lecithinen bzw. Derivaten der allgemeinen Formel I bzw. den reinen Diacylglycerinphosphatidylethanolaminen verwendet man auch Gemische dieser Stoffe, um damit einzelne erwünschte Eigenschaften zu verstärken. Durch die Zugabe sogenannter Phosphatidsäuren (es handelt sich dabei um Diacylglycerinphosphorsäureester mit 8 bis 24 Kohlenstoffatomen in der aliphatischen Acylgruppe) erzielt man eine gewisse Polarisierung in den Liposomen, was für die Inkorporierung polarer Moleküle sich vorteilhaft auswirkt.

Im allgemeinen werden zur Herstellung der Liposomen mit darin eingeschlossenen Allergenen die Diacylglycerinphosphatidylcholine bzw. deren Derivate der allgemeinen Formel I bzw. die Diacylglycerinphosphatidylethanolamine bzw. Gemische dieser Stoffe mit dem Cholesterin und Phosphatidsäuren im molaren Verhältnis von 1 zu 0,1 bis 0,4 zu 0,1 bis 0,4 gemischt. Die Menge der zugefügten Phosphatidsäuren richtet sich nach der Ladung des zu inkorporierenden Allergens. Die Lipide werden in einem Lösungsmittel, z.B. in Chloroform oder Methanol, gelöst, am Rotationsverdampfer wird das Lösungsmittel wieder entfernt. Danach gibt man einen isotonischen Phosphatpuffer, z.B. Dinatriumhydrogenphosphat/Natriumdihydrogenphosphat, zu, dem das Allergen vorher beigemischt wurde. Auf 1 mM des eingesetzten Lecithins oder Lecithinderivates fügt man im allgemeinen 15 bis 30 ml dieses allergenhaltigen isotonischen Phosphatpuffers, bei einer Allergenkonzentration von 0,1 bis 1,0 mg/ml, zu. In einer anderen Ausführungsform kann der Zusatz der Allergene auch nach der nachstehend erläuterten Druckbehandlung der Lipide erfolgen.

Bei der Herstellung der Liposomen, wird zur Einarbeitung hoher Cholesterinmengen das Gemisch aus den Lecithinen und dem Cholesterin hohen Drucken bis zu 1500 bar ausgesetzt und, gegebenenfalls erst anschließend ultrabeschallt. Mit Hilfe dieser Methode - auch French-Press-Methode genannt - erreicht man eine erleichterte Einarbeitung relativ hoher Cholesterinmengen und damit eine wünschenswert hohe Stabilität der Liposomen.

Für die Herstellung von Liposomen kommen vorzugsweise Stearyl- und Palmitylglycerinphosphatidylcholine in Frage, genau so gut eignen sich aber auch beispielsweise Dipelargonoyllecithin, Dicaprinoyllecithin und Dibehenyllecithin, neben einer ganzen Reihe weiterer, pharmakologisch verträglicher Lecithine.

Die Trennung der Liposomen von nichtinkorporierten Allergenen erfolgt in beiden Fällen vorteilhafterweise über Säulen, beispielsweise unter Benutzung von Sephadex-G® oder Sepharose 4B®. Sephadex-G® ist ein dreidimensional vernetztes Polysaccharid, das durch Quervernetzungen der linearen Makromoleküle von Dextran erhalten wird, es dient speziell der Gelchromatographie. Sepharose 4B $^R$ stellt ein Sortiment von

perlförmigen Materialien zur Gelchromatographie in wässriger Lösung auf Basis modifizierter Agarose dar, deren Polysaccharidketten zu einem dreidimensionalen Netzwerk verknüpft sind.

Die so erhaltenen Liposomen werden sterilisiert und bei einem Druck von 0,3 bis 2 Torr lyophilisiert. Für die oralen Darreichungsformen werden die Liposomen zum Schutz vor Magen- und Duodenalsaft in magensaftresistente Kapseln gefüllt.

Experimentelle Untersuchungen

Mit den nach Beispiel 5 hergestellten Liposomen wurde bei schwarzen Mäusen (Stamm CH3) untersucht, inwieweit diese Präparation einen immunogenen Reiz hinsichtlich der Entstehung von IgE-Antikörpern induziert. Bei dem Allergen handelt es sich um Dermatophagoides pteronyssinus-Antigen.

Methodik:

Folgende Vergleichsgruppen wurden gebildet:

A leere Liposomen
B Liposomen mit Allergen gefüllt gemäß der Verfahrensweise des Beispiels 5
C freies Allergen + Aluminiumhydroxid
D freies Allergen
E leere Liposomen
F leere Liposomen und Allergen nebeneinander

| | |
|---|---|
| Injektionsintervalle: | 1., 14., 25., 28. Tag |
| Blutabnahme: | 31. Tag |
| Dosis: | 30µg Allergen |
| | 9 µMol Lipid |
| Injektionsvolumen: | 200 µl |

Ergebnisse:

Bestimmung des spez. IgE-Titers

Die IgE-Titer wurden mittels PCA (passive kutane Anaphylaxie) in Mäusen bestimmt.
Den Mäusen wurden entsprechend dem oben erwähnten Schema die Allergene subkutan injiziert, am 31. Tag Blut abgenommen und aus diesem das Serum gewonnen.

50µl der Serumverdünnungen 1:1, 1:10, 1:30 und 1:90 wurden Ratten intradermal injiziert.
24 Stunden später wurden den Ratten 70µg des freien Allergens mit 0,5 ml 0,5%iger Evan's Blaulösung intravenös injiziert. Nach 30 Minuten wurden die Tiere getötet und die Reaktionsfläche an den Stellen gemessen, wo das Serum intradermal injiziert worden war.

Für die Gruppe A (leere Liposomen) und die Gruppe B (Liposomen mit Allergen gefüllt) konnte kein IgE nachgewiesen werden. Für die Gruppe C (Allergen + $Al(OH)_3$) konnte noch in einer Verdünnung von 1:30 und für die Gruppe D (freies Allergen) noch in einer Verdünnung 1:10 IgE nachgewiesen werden.

Abbildung 1: Rattenhaut zum Nachweis der passiven kutanen Anaphylaxie

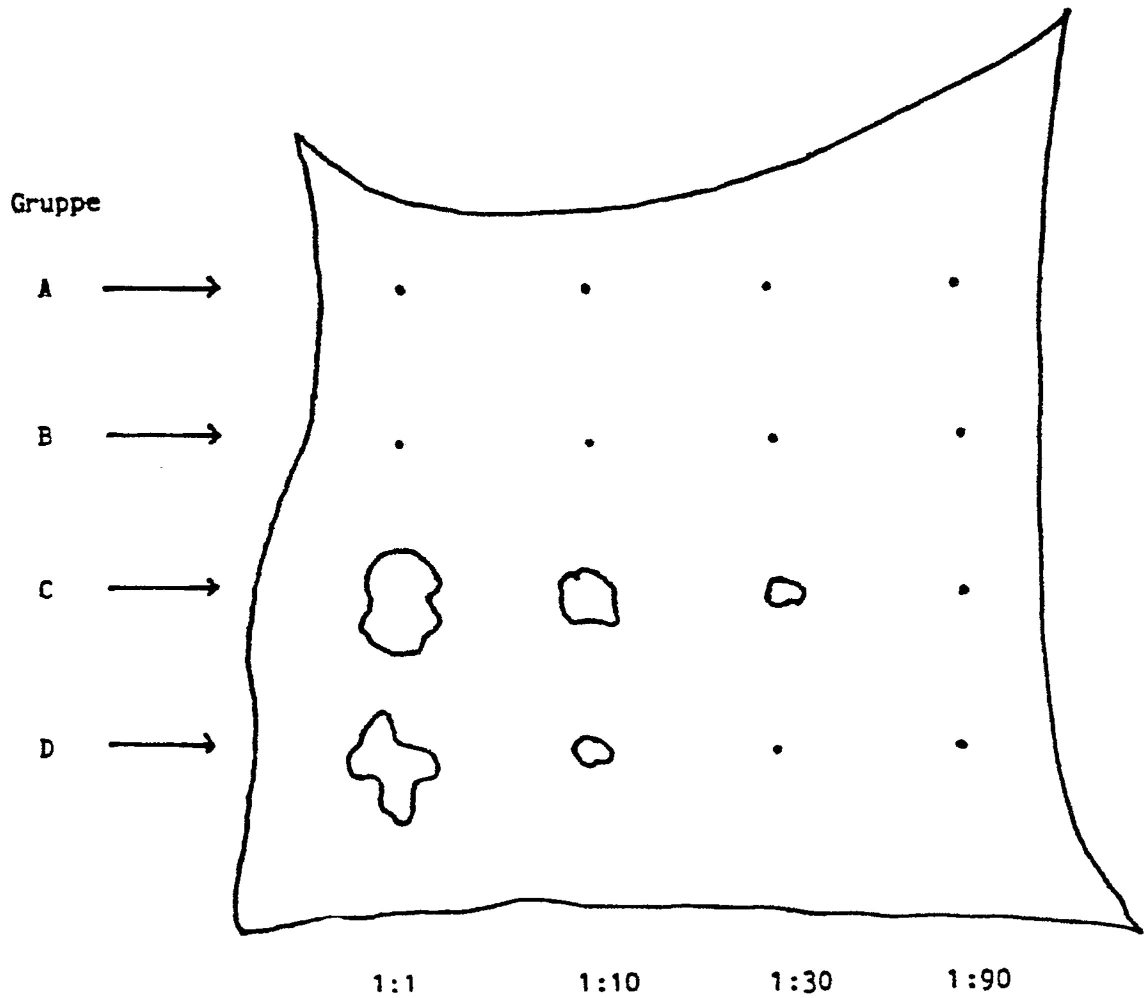


Bestimmung des IgG-Titers

Die Bestimmung des spez. IgG wurde radioimmunologisch durchgeführt.
PVC-Röhrchen wurden mit Dermatophagoides pteronsyssinus-Antigen beladen. Dazu wurden die Serumverdünnungen $10^{\circ}$, $10^{-1}$, $10^{-2}$, $10^{-3}$ und $10^{-4}$ gegeben. Anschließend wurde mit $^{125}$Jod-anti-Maus-IgG inkubiert, gewaschen und die Zählrate gemessen (Fig. 2).

Zusammenfassung:

Nach subkutaner Injektion von in Liposomen gemäß Beispiel 5 inkorporiertem Allergen konnten keine IgE, aber ein hoher Titer an spez. IgG festgestellt werden.

Die Injektion der gleichen Dosis mit freiem und an $Al(OH)_3$ absorbiertem Allergen induzierte dagegen hohe anaphylaktoide IgE-Titer und nur 1/9 (mit $Al(OH)_3$) des spez. IgG-Titers, der mit dem in Liposomen eingeschlossenem Allergen erreicht wurde.
IgE = anaphylactoide Antikörper
IgG = blockierende Antikörper

Im folgenden sollen einige Verfahren zur Herstellung von Liposomen beschrieben werden:

Beispiel

Dipalmitoylphosphatidylcholin wird mit Cholesterin im molaren Verhältnis 60:40 im Lösungsmittel Diethylether gelöst. Es wird soviel Diethylether verwendet, daß eine Konzentration von 50 µmol/ml an beiden Stoffen nicht überschritten wird. Nach Abdampfen des Lösungsmittels werden die Lipide über der Phasenumwandlungstemperatur bei 40 bis 45 °C getempert, mit der allergenhaltigen wässrigen Lösung

versetzt, einem Druck von 1400 at ausgesetzt und ultrabeschallt. Der Zusatz der Allergene kann auch nach der Druckbehandlung der Lipide erfolgen. Die Liposomen werden nun von dem nicht inkorporierten Allergen durch Filtration über Sephadex getrennt und entsprechend durch ein Filter mit der Porengröße 0,2 µm sterilfiltriert.

Die folgenden Beispiele beschreiben einige Arzneimittelzubereitungen:

Beispiel I Kapseln

Die gefriergetrocknete Liposomen werden zum Schutz vor Magen- und Duodenalsaft in magensaftresistente Kapseln abgefüllt. Die Liposomenmenge pro Kapsel richtet sich nach dem erzielten Sensibilisierungsgrad. Die pro Kapsel abgefüllten Liposomen enthalten je nach dem Sensibilisierungsgrad und der Art der betreffenden Allergene oder Allergengemische zwischen 10 und 10000 Protein Nitrogen Units (PNU). Im allgemeinen wird pro Tag eine Kapsel oral verabreicht.

Beispiel II Ampullen

Die Ampullen werden mit sterilen suspendierten oder lyophylisierten Liposomen beschickt. Die Liposomenmenge wird so eingestellt, daß entsprechend dem Sensibilisierungsgrad des Patienten und entsprechend der Art der Allergene bzw. Allergengemische zwischen 1 und 10000 PNU pro Ampulle enthalten sind.

## Patentansprüche

**1.** Liposomen enthaltend in ihren wässrigen Kompartimenten inhalative Allergene oder Allergenfraktionen in einem isotonen Phosphatpuffer, dadurch gekennzeichnet, daß sie erhalten werden aus

a) Diacylglycerinphosphatidylethanolaminen mit 8 bis 24 Kohlenstoffatomen in der aliphatischen Acylgruppe und/oder Lecitinen bzw. Lecithinderivaten der allgemeinen Formel

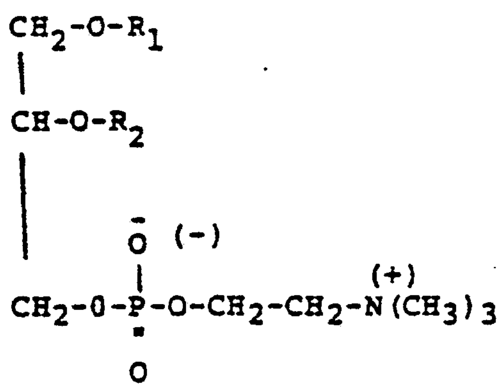

, (I)

in der $R_1$ und $R_2$ aliphatische Acylreste mit 8 bis 24 Kohlenstoffatomen darstellen, wobei $R_2$ aber auch eine Alkylgruppe mit 1 bis 8 Kohlenstoffatomen bedeuten kann, und in der die Gruppen $-OR_1$, $-OR_2$ und $-O-PO_3CH_2-CH_2-N(CH_3)_3$ gegeneinander vertauscht sein können, gegebenenfalls in Anwesenheit von Diacylglycerinphosphorsäureestern mit 8 bis 24 Kohlenstoffatomen im aliphatischen Acylrest und/oder von adjuvanten Lecithinen der allgemeinen Formel I, in der $R_1$ oder $R_2$ ein Wasserstoffatom ist und der andere dieser Reste $R_1$ bzw. $R_2$ einen aliphatischen Acylrest mit 8 bis 24 Kohlenstoffatomen oder einen Alkylrest mit 8 bis 24 Kohlenstoffatomen bedeutet, und

b) Cholesterin, wobei das Gemisch aus den Lecithinen und dem Cholesterin während der Herstellung der Liposomen einem Druck bis zu 1500 bar ausgesetzt wird.

**2.** Liposomen gemäß Anspruch 1, dadurch gekennzeichnet, daß die Diacylglycerinphosphatidylethanolamine und sonstigen Lecithine bzw. Lecithinderivate der allgemeinen Formel I und, gegebenenfalls die adjuvanten Lecitihine, die einzeln oder miteinander vermischt sein können, mit dem Cholesterin in einem molaren Verhältnis von 1 zu 0,1 bis 0,4 vorliegen.

**3.** Liposomen gemäß Anspruch 1 und 2, dadurch gekennzeichnet, daß die Diacylglycerinphosphatidylethanolamine und sonstigen Lecithine bzw. Lecithinderivate der allgemeinen Formel I und, gegebenenfalls

die adjuvanten Lecithine, die einzeln oder miteinander vermischt sein können, mit dem Cholesterin und einem Diacylglycerinphosphorsäureester mit 8 bis 24 Kohlenstoffatomen im aliphatischen Diacylrest in einem molaren Verhältnis von 1 zu 0,1 bis 0,4 zu 0,1 bis 0,4 vorliegen.

**4.** Liposomen gemäß Anspruch 1, 2 und 3, dadurch gekennzeichnet, daß die Liposomen auf 1 mM des eingesetzten Diacylglycerinphosphatidylethanolamins bzw. Lecithins bzw. Lecithinderivats der Formel I, 15 bis 30 ml einer allergenhaltigen, isotonen wässrigen Phosphatpufferlösung mit einer Konzentration an Allergenen oder Allergenfraktionen von 0,1 bis 1 mg pro ml Phosphatpufferlösung enthalten.

**5.** Arzneimittel enthaltend Liposomen mit darin inkorporierten inhalativen Allergenen oder Allergenfraktionen nach einem der Ansprüche 1 bis 4.

**6.** Verfahren zur Herstellung von inhalative Allergene oder Allergenfraktionen enthaltende Liposomen, dadurch gekennzeichnet, daß Diacylglycerinphosphatidylethanolamine mit 8 bis 24 Kohlenstoffatomen in der aliphatischen Acylgruppe und/oder sonstige Lecithine bzw. Lecithinderivate der allgemeinen Formel I

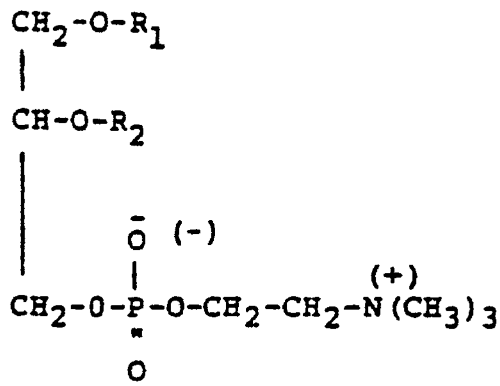

, (I)

in der $R_1$ und $R_2$ aliphatische Acylreste mit 8 bis 24 Kohlenstoffatomen darstellen, wobei $R_2$ aber auch eine Alkylgruppe mit 1 bis 8 Kohlenstoffatomen bedeuten kann, und in der die Gruppen $-OR_1$, $-OR_2$ und $-O-PO_3CH_2-CH_2-N(CH_3)_3$ gegeneinander vertauscht sein können, gegebenenfalls aber auch Diacylglycerinphosphorsäureester mit 8 bis 24 Kohlenstoffatomen im aliphatischen Diacylrest und/oder adjuvante Lecithine der allgemeinen Formel I, in der $R_1$ oder $R_2$ ein Wasserstoffatom ist und der andere dieser Reste $R_1$ bzw. $R_2$ einen aliphatischen Acylrest mit 8 bis 24 Kohlenstoffatomen oder einen Alkylrest mit 8 bis 24 Kohlenstoffatomen bedeutet, mit Cholesterin vermischt und in einem Lösungsmittel gelöst werden, das Lösungsmittel wieder entfernt, der Rückstand mit einem isotonen Phosphatpuffer versetzt und das Gemisch einem Druck bis zu 1500 bar ausgesetzt wird, wobei die Allergene oder Allergenfraktionen entweder in dem isotonen Phosphatpuffer enthalten sind oder erst nach der Druckbehandlung der Lipide zugesetzt werden und daß nicht inkorporierte Allergene entfernt und die gereinigten Liposomen sterilisiert und gegebenenfalls lyophilisiert werden.

**7.** Verfahren gemäß Anspruch 6, dadurch gekennzeichnet, daß die Diacylglycerinphosphatidylethanolamine und/oder Lecithine bzw. Lecitinderivate der allgemeinen Formel I mit dem Cholesterin im molaren Verhältnis von 1 zu 0,1 bis 0,4 vermischt werden und nach dem Abdampfen des Lösungsmittels auf 1 mM des eingesetzten Diacylglycerinphosphatidylethanolamins bzw. Lecithins bzw. Lecithinderivates der allgemeinen Formel I bzw. eines Gemisches dieser Komponenten 15 bis 30 ml eines allergenhaltigen isotonischen Phosphatpuffers, der eine Allergenkonzentration von 0,1 bis 1,0 mg pro ml Pufferlösung aufweist, zugemischt werden.

**8.** Verfahren gemäß Anspruch 6 und 7, dadurch gekennzeichnet, daß man zu der Lecithinkomponente noch einen Diacylglycerinphosphorsäureester im molaren Verhältnis von 1 zu 0,1 bis 0,4 zumischt.

**9.** Verwendung von Liposomen gemäß den Ansprüchen 1 bis 4 zur Einbettung inhalativer Allergenen oder Allergenfraktionen in ihre wässrigen Kompartimente für die subkutane, inhalative oder orale Applikation.

## Claims

1. Liposomes containing in their aqueous compartments inhalative allergens or fractions of allergens in isotonic phosphate buffer, characterized in that they are obtained from
   a) diacyl glycerol phosphatidyl ethanol amines having 8 to 24 carbon atoms in the aliphatic acyl group and/or lecithins or lecithin derivatives of the general formula

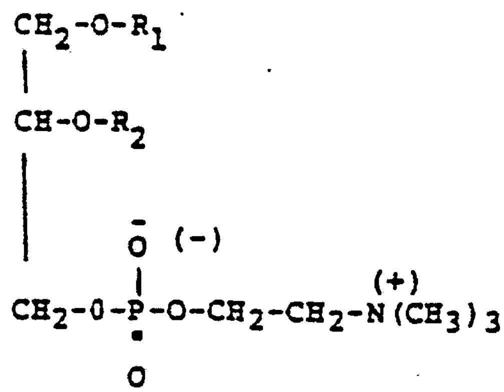

, (I)

   wherein $R_1$ and $R_2$ represent aliphatic acyl groups having 8 to 24 carbon atoms, with $R_2$ optionally being an alkyl group having 1 to 8 carbon atoms, and wherein $-OR_1$, $-OR_2$ and $-O-PO_3CH_2-CH_2-N-(CH_3)_3$ are interchangeable, if desired, in the presence of diacyl glycerol phosphoric esters having 8 to 24 carbon atoms in the aliphatic acyl group and/or of adjuvant lecithins of general formula I wherein one of $R_1$ and $R_2$ is hydrogen and the other of said groups $R_1$ and $R_2$ is an aliphatic acyl group having 8 to 24 carbon atoms or an alkyl group having 8 to 24 carbon atoms, and
   b) cholesterol,
   with the mixture of said lecithins and said cholesterol being exposed to a pressure of up to 1,500 bar during the production of said liposomes.

2. Liposomes as defined in claim 1, characterized in that said diacyl glycerol phosphatidyl ethanol amines and other lecithins or lecithin derivatives of general formula I and, if desired, said adjuvant lecithins, which may be mixed separately or together, plus said cholesterol are present in a molar ratio of 1 : 0.1 to 0.4.

3. Liposomes as defined in claims 1 and 2, characterized in that said diacyl glycerol phosphatidyl ethanol amines and other lecithins or lecithin derivatives of general formula I and, if desired, said adjuvant lecithins, which may be mixed separately or together, plus said cholesterol and a diacyl glycerol phosphoric ester having 8 to 24 carbon atoms in the aliphatic diacyl group are present in a molar ratio of 1 : 0.1 to 0.4 : 0.1 to 0.4.

4. Liposomes as defined in claims 1, 2 and 3, characterized in that said liposomes contain, per 1 mmole of diacyl glycerol phosphatidyl ethanol amine or lecithin or lecithin derivative of formula I used, 15 to 30 ml of allergen-containing, isotonic aqueous phosphate buffer solution having a concentration of allergens or allergen fractions of 0.1 to 1 mg per ml of phosphate buffer solution.

5. A pharmaceutical composition containing liposomes as defined in claims 1 to 4 having inhalative allergens or fractions of allergens incorporated therein.

6. A method for producing inhalative allergens or fractions of allergens containing liposomes, characterized by mixing diacyl glycerol phosphatidyl ethanol amines having 8 to 24 carbon atoms in the aliphatic acyl group and/or other lecithins or lecithin derivatives of general formula I

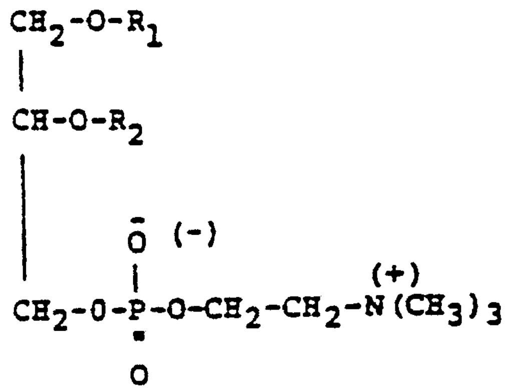

, (I)

wherein $R_1$ and $R_2$ represent aliphatic acyl groups having 8 to 24 carbon atoms, with $R_2$ optionally being an alkyl group having 1 to 8 carbon atoms, and wherein $-OR_1$, $-OR_2$ and $-O-PO_3CH_2-CH_2-N-(CH_3)_3$ are interchangeable, and, if desired, also diacyl glycerol phosphoric esters having 8 to 24 carbon atoms in the aliphatic diacyl group and/or adjuvant lecithins of general formula I wherein one of $R_1$ and $R_2$ is hydrogen and the other of said groups $R_1$ and $R_2$ is an aliphatic acyl group having 8 to 24 carbon atoms or an alkyl group having 8 to 24 carbon atoms, with cholesterol and dissolving the mixture in a solvent, removing the solvent again, supplementing the residue with isotonic phosphate buffer and exposing the mixture to a pressure of up to 1,500 bar, with the allergens or allergen fractions either being contained in the isotonic phosphate buffer or being added after exposure of the lipids to pressure, and by removing non-incorporated allergens and by sterilizing and, if desired, lyophilizing the purified liposomes.

**7.** A method as defined in claim 6, characterized by mixing said diacyl glycerol phosphatidyl ethanol amines and/or lecithins or lecithin derivatives of general formula I with said cholesterol in a molar ratio of 1 : 0,1 to 0,4 and, after evaporating the solvent, adding thereto, per 1 mmole of diacyl glycerol phosphatidyl ethanol amine or lecithin or lecithin derivative of general formula I or a mixture of these components used, 15 to 30 ml of allergen-containing, isotonic phosphate buffer having an allergen concentration of 0.1 to 1.0 mg per ml of buffer solution.

**8.** A method as defined in claims 6 and 7, characterized by also adding to the lecithin component a diacyl glycerol phosphoric ester in a molar ratio of 1 : 0.1 to 0.4.

**9.** Use of liposomes as defined in claims 1 to 4 for embedding inhalative allergens or fractions of allergens in their aqueous compartments for subcutaneous, inhalative or oral application.

## Revendications

**1.** Liposomes, contenant dans leurs compartiments aqueux des allergènes ou fractions d'allergène à inhaler dans un tampon de phosphate isotonique, caractérisés en ce qu'ils sont obtenus à partir

a) de diacylglycérinephosphatidyléthanolamines comportant 8 à 24 atomes de carbone dans le groupe acyle aliphatique et/ou de lécithines et respectivement de dérivés de lécithine répondant a la formule générale

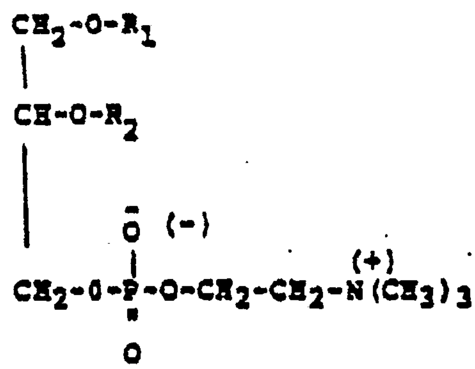

, (I)

dans laquelle $R_1$ et $R_2$ représentent des radicaux acyle aliphatiques ayant 8 à 24 atomes de carbone, $R_2$ pouvant cependant également représenter un groupe alkyle ayant 1 à 8 atomes de carbone, et dans laquelle les groupes $-OR_1$ $-OR_2$ et $-O-PO_3CH_2-CH_2-N(CH_3)_3$ peuvent être échangés entre eux, éventuellement en présence d'esters d'acide diacylglycérinephosphorique comportant 8 à 24 atomes de carbone dans le radical acyle aliphatique et/ou de lécithines additionnelles répondant à la formule générale I, dans laquelle $R_1$ ou $R_2$ représente un atome d'hydrogène et l'autre de ces radicaux $R_1$ et respectivement $R_2$ un radical acyle aliphatique comportant 8 à 24 atomes de carbone ou un radical alkyle comportant 8 à 24 atomes de carbone, et

b) de cholestérol,

le mélange des lécithines et du cholestérol étant, pendant la préparation des liposomes, soumis à une pression allant jusqu'à 1.500 bars.

2. Liposomes suivant la revendication 1, caractérisés en ce que les diacylglycérinephosphatidyléthanolamines et autres lécithines et respectivement dérivés de lécithine de la formule générale I et éventuellement les lécithines additionnelles, qui peuvent être mélangées isolément ou entre elles, sont au cholestérol dans un rapport molaire de 1 pour 0,1 à 0,4.

3. Liposomes suivant l'une des revendications 1 et 2, caractérisés en ce que les diacylglycérinephosphatidyléthanolamines et autres lécithines ou respectivement dérivés de lécithine de la formule générale I et éventuellement les lécithines additionnelles, qui peuvent être mélangées isolément ou entre elles, sont au cholestérol et à un ester d'acide diacylglycérinephosphorique comportant 8 à 24 atomes de carbone dans le radical diacyle aliphatique dans un rapport molaire de 1 pour 0,1 à 0,4 et pour 0,1 à 0,4.

4. Liposomes suivant l'une des revendications 1, 2 et 3, caractérisés en ce que les liposomes contiennent pour 1 mM de la diacylglycérinephosphatidyléthanolamine ou respectivement de la lécithine ou respectivement du dérivé de lécithine de la formule I mis en oeuvre 15 à 30 ml d'une solution de tampon de phosphate aqueuse isotonique, contenant de l'allergène et présentant une concentration en allergènes ou fractions d'allergène de 0,1 à 1 mg par ml de solution de tampon de phosphate.

5. Médicament contenant des liposomes comportant des allergènes ou fractions d'allergène à inhaler, incorporés, selon l'une des revendications 1 à 4.

6. Procédé de préparation de liposomes contenant des allergènes ou fractions d'allergène à inhaler, caractérisé en ce que des diacylglycérinephosphatidyléthanolamines comportant 8 à 24 atomes de carbone dans le groupe acyle aliphatique et/ou d'autres lécithines ou respectivement dérivés de lécithine répondant à la formule générale I

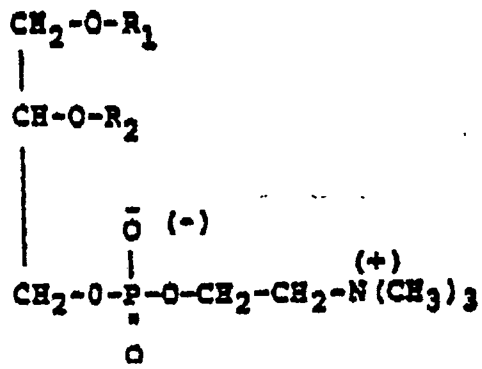

, (I)

dans laquelle $R_1$ et $R_2$ représentent des radicaux acyle aliphatiques ayant 8 à 24 atomes de carbone, $R_2$ pouvant cependant également représenter un groupe alkyle ayant 1 à 8 atomes de carbone, et dans laquelle les groupes $-OR_1$, $-OR_2$ et $-O-PO_3CH_2-CH_2-N(CH_3)_3$ peuvent être mutuellement échangés, et éventuellement aussi des esters d'acide diacylglycérinephosphorique ayant 8 à 24 atomes de carbone dans le radical diacyle aliphatique et/ou des lécithines additionnelles de la formule générale I, dans laquelle $R_1$ ou $R_2$ représente un atome d'hydrogène et l'autre de ces radicaux $R_1$ et respectivement $R_2$ un radical acyle aliphatique ayant 8 à 24 atomes de carbone ou un radical alkyle ayant 8 à 24 atomes de carbone, sont mélangés à du cholestérol et sont dissous dans un solvant, en ce que le solvant est à nouveau éliminé, en ce qu'on ajoute au résidu un tampon de phosphate isotonique et en ce qu'on soumet le mélange à une pression allant jusqu'à 1.500 bars, les allergènes ou fractions d'allergène étant contenus dans le tampon de phosphate isotonique ou étant ajoutés uniquement après le traitement sous pression des lipides, et en ce qu'on élimine les allergènes non incorporés et on stérilise et éventuellement lyophilise les liposomes purifiés.

**7.** Procédé suivant la revendication 6, caractérisé en ce que les diacylglycérinephosphatidyléthanolamines et/ou lécithines et respectivement dérivés de lécithine de la formule générale I sont mélangés au cholestérol dans un rapport molaire de 1 pour 0,1 à 0,4 et en ce qu'après l'évaporation du solvant, à 1 mM de la diacylglycérinephosphatidyléthanolamine ou de la lécithine ou du dérivé de lécithine de la formule générale I mis en oeuvre ou respectivement d'un mélange de ces composants, on ajoute 15 à 30 ml d'un tampon de phosphate isotonique contenant de l'allergène qui présente une concentration d'allergène de 0,1 à 1,0 mg par ml de solution tampon.

**8.** Procédé suivant l'une des revendications 6 et 7, caractérisé en ce qu'on ajoute encore au composant de lécithine un ester d'acide diacylglycérinephosphorique dans un rapport molaire de 1 pour 0,1 à 0,4.

**9.** Utilisation de liposomes suivant l'une des revendications 1 à 4 pour l'incorporation d'allergènes ou fractions d'allergène à inhaler dans leurs compartiments aqueux, en vue de l'application sous-cutanée, par inhalation ou par voie orale.

Figur 1

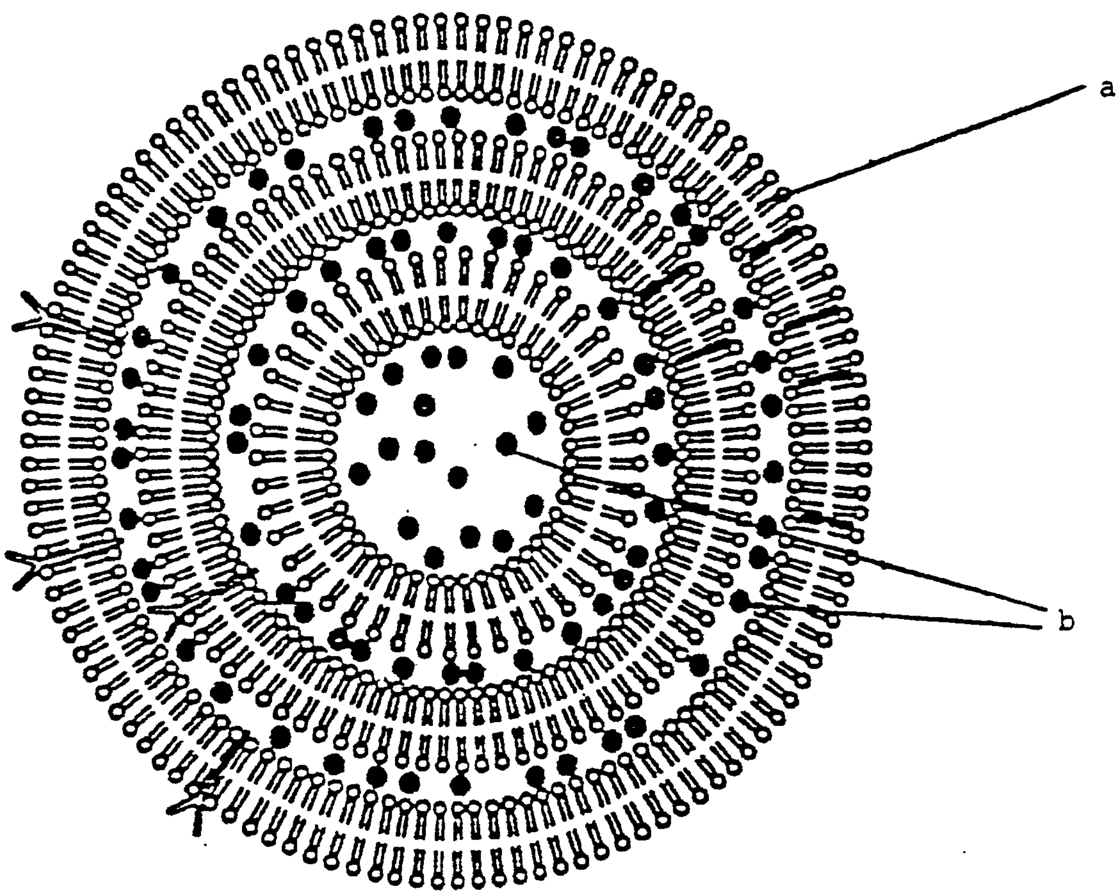

Figur 2

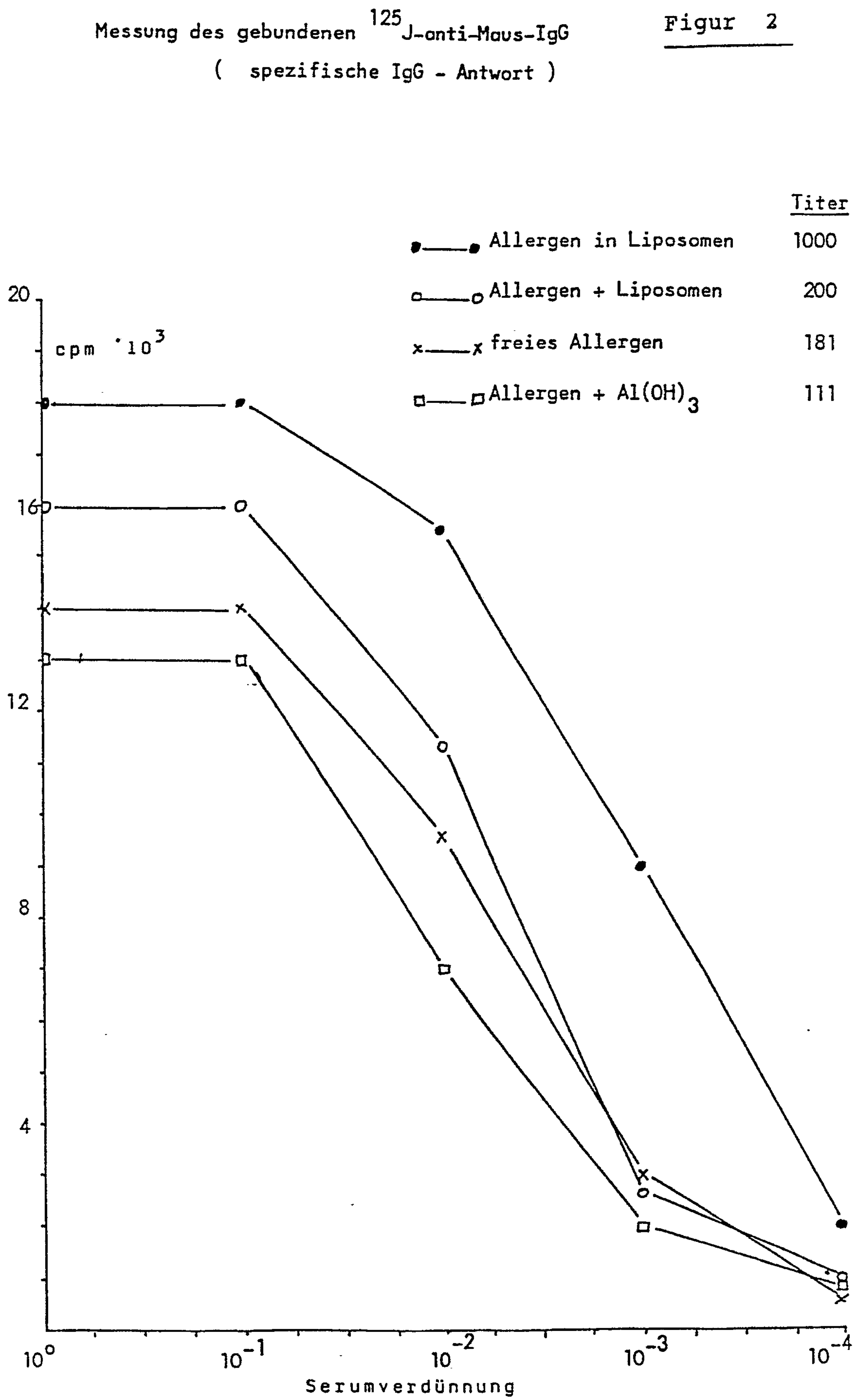
Messung des gebundenen $^{125}$J-anti-Maus-IgG
( spezifische IgG - Antwort )
Titer
Allergen in Liposomen 1000
Allergen + Liposomen 200
freies Allergen 181
Allergen + Al(OH)3 111
cpm ·10³
20
16
12
8
4
10⁰
10⁻¹
10⁻²
10⁻³
10⁻⁴
Serumverdünnung